# EUROPEAN PATENT SPECIFICATION

(11) **EP 4 100 432 B1**
(45) Date of publication and mention of the grant of the patent: **19.03.2025**
(21) Application number: 21704034.4
(22) Date of filing: 03.02.2021
(51) Int. Cl.: C07K 14/765, A23L 13/00, C12N 5/00, C12N 5/077, C12N 5/0775

(54) **SERUM-FREE MEDIUM FOR CULTURING A BOVINE PROGENITOR CELL**
SERUMFREIES MEDIUM ZUR KULTIVIERUNG EINER VORLÄUFERZELLE
MILIEU EXEMPT DE SÉRUM POUR LA CULTURE D'UNE CELLULE PROGÉNITRICE

(30) Priority: 03.02.2020 EP 20386007
(43) Date of publication of application: 14.12.2022
(73) Proprietor: Mosa Meat B.V., 6229 PM Maastricht (NL)
(72) Inventor: MOUTSATSOU, Panagiota, 6229 PM Maastricht (NL); CRUZ, Helder, 6229 PM Maastricht (NL); KLEVERNIC, Iva, 6229 PM Maastricht (NL); KOLKMANN, Anna, 6229 PM Maastricht (NL); VAN ESSEN, Anon, 6229 PM Maastricht (NL); POST, Mark, 6229 PM Maastricht (NL)
(74) Representative: V.O.
(86) International application number: PCT/NL2021/050066
(87) International publication number: WO 2021/158103

(56) References cited:
- EP-A1- 1 988 159
- EP-A1- 2 351 831
- WO-A1-00/27996
- WO-A1-2010/031190
- WO-A2-2018/011805
- CN-A- 105 112 364
- JÉROME CHAL ET AL: "Differentiation of pluripotent stem cells to muscle fiber to model Duchenne muscular dystrophy", NATURE BIOTECHNOLOGY, vol. 33, no. 9, 3 August 2015 (2015-08-03), New York, pages 962 - 969, XP055281323, ISSN: 1087-0156, DOI: 10.1038/nbt.3297
- THORREZ LIEVEN ET AL: "Challenges in the quest for 'clean meat'", NATURE BIOTECHNOLOGY, GALE GROUP INC., NEW YORK, US, vol. 37, no. 3, 4 March 2019 (2019-03-04), pages 215 - 216, XP036900611, ISSN: 1087-0156, [retrieved on 20190304], DOI: 10.1038/S41587-019-0043-0

## Description

### FIELD OF THE INVENTION

The invention is in the field of serum-free media for animal cell culture, more specifically a serum-free medium for use in methods of culturing bovine progenitor cells, including (skeletal) muscle tissue-derived progenitor cells and adipose tissue-derived progenitor cells. The invention also relates to methods of culturing bovine progenitor cells using such a culture medium.

### BACKGROUND TO THE INVENTION

Serum from varied origins has been used as an essential component in animal cell culture media, since it provides several important nutrients, vitamins, growth factors and adhesion proteins, among other components. However, serum is a potential source of contaminants such as bacteria, mycoplasma, viruses, and prions, with the latter being a more recent source of concern since they are agents of transmissible neurodegenerative diseases in humans and other animals, from which serum is most often obtained, such as from bovines. These considerations play an important role, especially when the animal cell types are cultured for food applications, such as cell culture-based meat production for human consumption.

Moreover, serum represents a high cost to the bioprocesses and introduces variability in performance given the lot to lot variation of sera. The animal well-being is becoming an additional source of concern if serum is used in cell culture applications.

Therefore, there is a need to avoid the use of serum in cell culture applications (i.e. using a serum-free medium), while still achieving an acceptable performance in terms of cell proliferation - such as cell growth rate or longevity (total number of cell doublings) -which is preferably comparable with that of a serum-containing medium.

Especially for cell culture-based meat production for human consumption, there is a need for a serum-free medium that allows for proliferation, not differentiation, of bovine muscle tissue-derived and bovine adipose tissue-derived progenitor cells. Such expanded progenitor cell population can subsequently be cultured in a differentiation medium for differentiating said progenitor cells into muscle cells and muscle fibers or adipose tissue cells that can be incorporated into a cell-culture based meat product for human consumption. Unfortunately, the current offer in serum-free media for proliferation of bovine progenitor cells is limited, and the performance of available culture media is often unsatisfactory.

### SUMMARY OF THE INVENTION

The present inventors discovered a serum-free medium that can be used for culture and proliferation (expansion) of non-human, mammalian progenitor cells, especially bovine progenitor cells such as bovine muscle tissue-derived progenitor cells and bovine adipose tissue-derived progenitor cells.

Therefore, the invention provides in a first aspect a method for culturing a bovine progenitor cell, comprising the step of: culturing a bovine progenitor cell in a serum-free medium for culturing a bovine progenitor cell, wherein said serum-free medium comprises - an albumin; - a fibroblast growth factor 2 (FGF2); - an interleukin-6 (IL-6); - an insulin-like growth factor 1 (IGF1); and - an hepatocyte growth factor (HGF)..

Also disclosed herein is a serum-free medium for culturing a bovine progenitor cell, comprising - an albumin; and - a fibroblast growth factor (FGF).

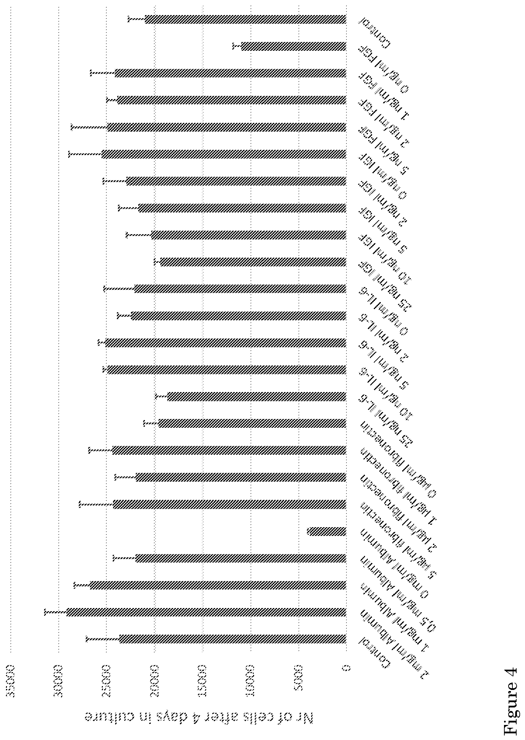

One of the findings of the present inventors is that bovine progenitor cells are strongly dependent on the presence of albumin and fibroblast growth factor when cultured serum-free for proliferation (expansion) purposes. Figure 4 shows that the absence of albumin and FGF in a serum-free medium strongly affects bovine progenitor cell proliferation rates. This is a new insight.

Also disclosed herein is a serum-free medium for culturing a bovine progenitor cell, comprising - albumin; and - a growth factor and/or a cytokine as described herein, preferably a fibroblast growth factor (FGF).

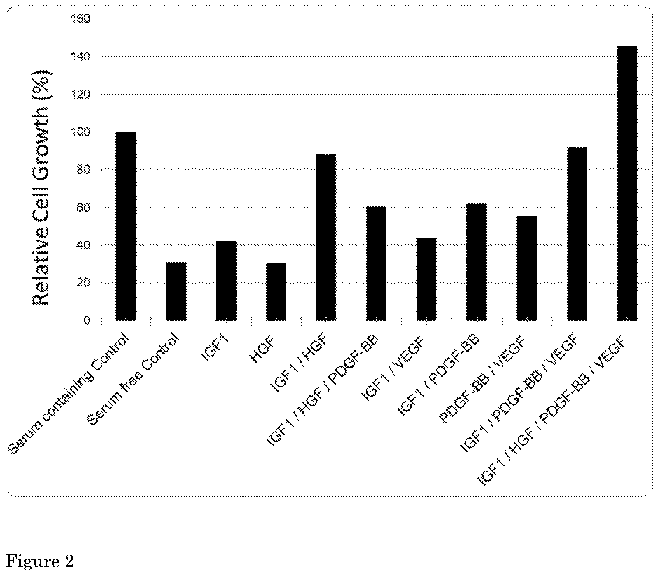

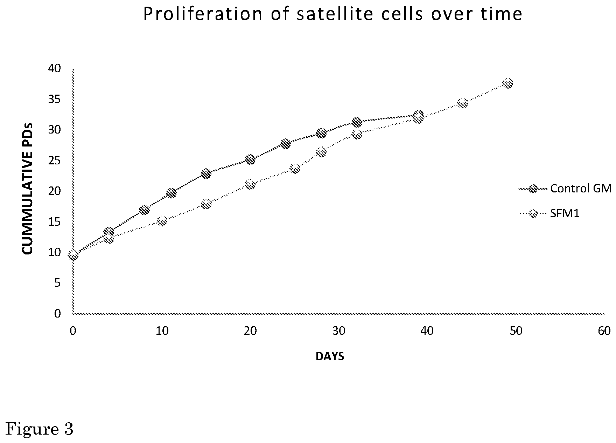

Another important achievement of the inventors was that they were able to chemically define a serum-free culture medium that can be used to proliferate progenitor cells, especially bovine progenitor cells. Advantages of a serum-free medium as disclosed herein are that it does not contain serum and is preferably also animal component-free. Such a serum-free culture medium can be produced at significantly lower costs than culture media that contain serum, which is a requirement in order to obtain a viable cell culture-based meat product. Moreover, it was unexpectedly established that the growth (curve) of bovine progenitor cells in a serum-free medium as disclosed herein is more stable than the growth (curve) of said cells in a serum-containing medium, since the growth rates in the latter are gradually decreasing until cell growth stops (see Figure 3). Figure 3 also shows that the growth rates of bovine progenitor cells in a serum-free medium as disclosed herein is advantageous, and are at least comparable to the ones achieved using a serum-containing medium. The experimental data further shows that bovine progenitor cells, which are used for cell culture-based meat production for human consumption, show good performance in a range of concentrations of media components (Figures 2 and 4).

Preferably, the medium further comprises: - one or more vitamins and/or hormones selected from the group consisting of ascorbic acid or a derivative thereof, an insulin, a somatotropin and a hydrocortisone.

Preferably, the medium further comprises - a basal medium; - a source of glucose (which can be provided in the form of (i.e. can be comprised in) said basal medium); - a source of glutamine (which can be provided in the form of (i.e. can be comprised in) said basal medium); - a source of fatty acids (which can be provided in the form of (i.e. can be comprised in) said basal medium); - a source of iron or an iron transporter (which can be provided in the form of (i.e. can be comprised in) said basal medium); and - sodium selenite (which can be provided in the form of (i.e. can be comprised in) said basal medium). Alternatively, the medium may further comprises one or more of - a basal medium; - a source of glucose (which can be provided in the form of (i.e. can be comprised in) said basal medium); - a source of glutamine (which can be provided in the form of (i.e. can be comprised in) said basal medium); - a source of fatty acids (which can be provided in the form of (i.e. can be comprised in) said basal medium); - a source of iron or an iron transporter (which can be provided in the form of (i.e. can be comprised in) said basal medium); and - sodium selenite (which can be provided in the form of (i.e. can be comprised in) said basal medium). Preferably, said serum-free medium comprises at least a basal medium, and preferably also - a source of glucose, - a source of glutamine, and - a source of fatty acids; and preferably in addition thereto - a source of iron or an iron transporter and/or - sodium selenite.

Preferably, a serum-free medium as disclosed herein comprises a basal medium which may already comprise a source of glucose and/or a source of glutamine; and may optionally also comprise a source of fatty acids; a source of iron or an iron transporter; and/or sodium selenite.

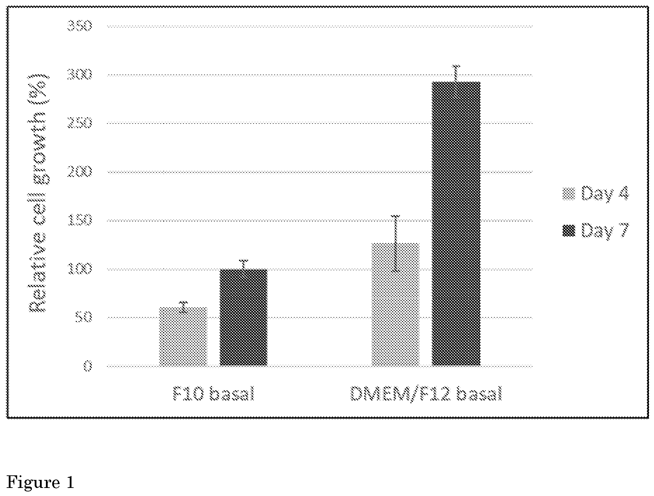

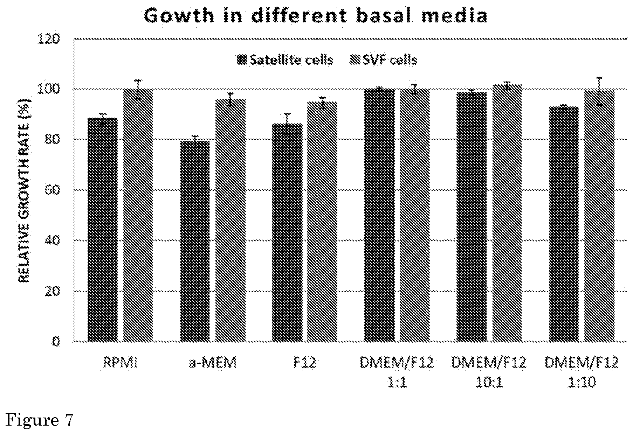

Preferably, when a basal medium is present, the basal medium comprises DMEM and/or Ham's F12 medium, preferably DMEM and Ham's F12 medium, for instance in a ratio of 1:10 to 10:1, more preferably in a 1:1 ratio, respectively. Figure 1 shows that a basal medium comprising both DMEM and Ham's F12 medium performs particularly well. Figure 7 shows that a basal medium comprising RPMI or alpha-MEM also performs well, which is also true for a basal medium comprising DMEM/F12 when provided in different ratios.

Preferably, when a source of fatty acids is present, said source of fatty acids comprises α-linolenic acid.

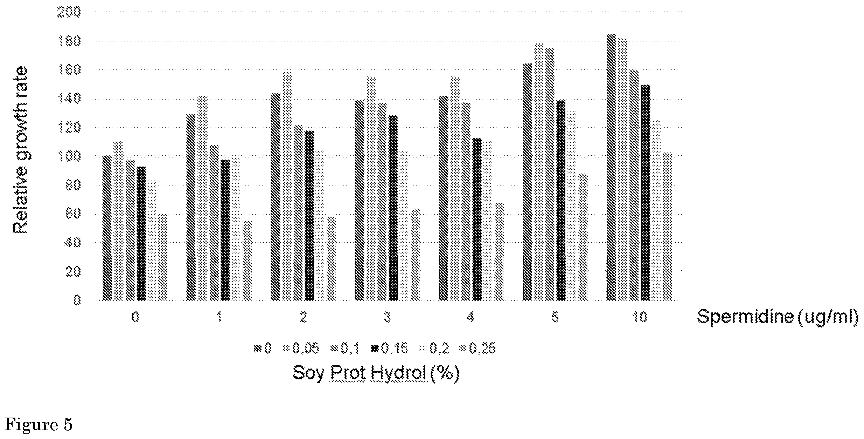

Preferably, said medium further comprises a protein hydrolysate, preferably a soy protein hydrolysate. It was unexpectedly established that the addition of a protein hydrolysate, such as a soy protein hydrolysate, to a serum-free growth medium provides for improved bovine progenitor cell growth rates (Figure 5).

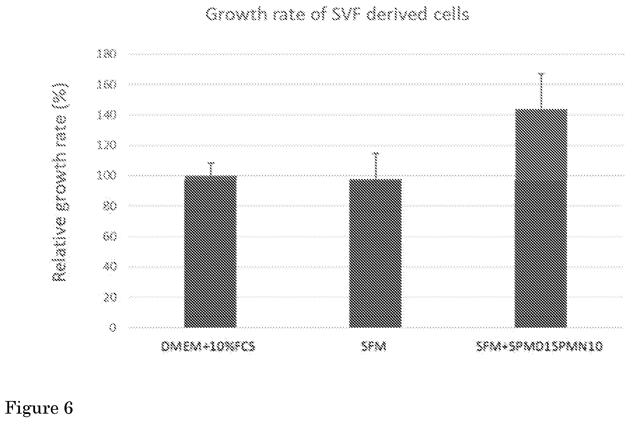

Preferably, said medium further comprising a biogenic amine, preferably a biogenic monoamine or polyamine, preferably an ethanolamine, a putrescine, a spermidine and/or a spermine. It was unexpectedly found that the addition of biogenic amines provides for improved bovine progenitor cell growth rates (Figures 5 and 6). Similarly, it was found that when used in combination, biogenic amines improved growth of adipose tissue-derived progenitor cells (Figure 6). Preferably, one or more biogenic amines are present in a serum-free medium as disclosed herein, more preferably at least two biogenic amines, such as at least spermine and spermidine.

Preferably, said medium further comprises one or more attachment factors.

Preferably, when said medium comprises an attachment factor, said attachment factor is fibronectin.

A serum free medium as disclosed herein may comprise: - an albumin; - a fibroblast growth factor (FGF); - one or more vitamins and/or hormones selected from the group consisting of ascorbic acid or a derivative thereof, an insulin, a somatotropin and a hydrocortisone; - one or more cytokines and/or growth factors selected from the group consisting of a platelet-derived growth factor (PDGF), an insulin-like growth factor (IGF), a vascular endothelial growth factor (VEGF), an hepatocyte growth factor (HGF) and an interleukin 6 (IL-6); - a basal medium; - a source of glucose (which can be provided in the form of (i.e. can be comprised in) said basal medium); - a source of glutamine (which can be provided in the form of (i.e. can be comprised in) said basal medium); - a source of fatty acids (which can be provided in the form of (i.e. can be comprised in) said basal medium); - a source of iron or an iron transporter (which can be provided in the form of (i.e. can be comprised in) said basal medium); and - sodium selenite (which can be provided in the form of (i.e. can be comprised in) said basal medium); and optionally a protein hydrolysate, a biogenic amine and/or an attachment factor.

Preferably, the serum free medium comprises: - an albumin; - a fibroblast growth factor (FGF); - ascorbic acid or a derivative thereof, an insulin, a somatotropin and a hydrocortisone; - a platelet-derived growth factor (PDGF), an insulin-like growth factor (IGF), a vascular endothelial growth factor (VEGF), an hepatocyte growth factor (HGF) and an interleukin 6 (IL-6); - a basal medium; - a source of glucose (which can be provided in the form of (i.e. can be comprised in) said basal medium); - a source of glutamine (which can be provided in the form of (i.e. can be comprised in) said basal medium); - a source of fatty acids (which can be provided in the form of (i.e. can be comprised in) said basal medium); - a source of iron or an iron transporter (which can be provided in the form of (i.e. can be comprised in) said basal medium); and - sodium selenite (which can be provided in the form of (i.e. can be comprised in) said basal medium); and optionally a protein hydrolysate, a biogenic amine and/or an attachment factor.

Preferably, said albumin, said FGF, said one or more cytokines and/or growth factors, said one or more vitamins and/or hormones, said basal medium, said source of glucose, said source of glutamine, said source of fatty acids, said source of iron or said iron transporter, said sodium selenite, said protein hydrolysate, said biogenic amine and/or said attachment factor are present in an effective amount for proliferation of a bovine progenitor cell in culture.
Preferably, said medium further comprises one or more vitamins and/or hormones, preferably one or more of ascorbic acid (or a derivative thereof such as L-ascorbic acid 2-phosphate), insulin, somatotropin, and hydrocortisone.

Preferably, said medium further comprising a source of glucose, a source of glutamine, and/or a source of iron or an iron transporter. Preferably, a serum-free medium as disclosed herein comprises a source of glucose and a source of glutamine.

Preferably, said medium comprises a basal medium, such as a liquid basal medium, which may comprise a source of glucose and/or a source of glutamine; and may optionally also comprise a source of fatty acids; a source of iron or an iron transporter; and/or sodium selenite.

Preferably, when said medium comprises a source of glucose and a source of glutamine, said source of glutamine comprises glutamine or L-alanyl-L-glutamine, and/or said source of glucose comprises glucose.

Preferably, when said medium comprises an iron transporter, said iron transporter is a transferrin.

Preferably, the albumin is a human albumin, preferably a human albumin that is recombinantly produced.

Preferably, all the components of the medium are animal-free.

Preferably, when a basal medium is present in said medium, the basal medium comprises DMEM and/or Ham's F12 medium, preferably DMEM and Ham's F12 medium, for instance in a ratio of 1:10 to 10:1, more preferably in a 1:1 ratio, respectively. Alternatively, the basal medium may comprise RPMI or alpha-MEM.

Preferably, when a source of fatty acids is present in said medium, said source of fatty acids comprises α-linolenic acid.

Preferably, when a source of glucose is present in said medium, said source of glucose is present in a concentration of 0.01 - 75 g/l, more preferably 0.1 - 4.5 g/l.

Preferably, said FGF is present in a concentration of 0.1 - 1000 µg/l, preferably 1-100 µg/l, more preferably 5 - 50 pg/l, even more preferably about 10 µg/l.

Preferably, when a source of glutamine is present in said medium, said source of glutamine is present in a concentration of 0.01 - 100 mM, more preferably 0.1 - 8 mM, such as about 2mM.

Preferably, said albumin is present in a concentration of 0.01 - 50 g/l, preferably 0.1 - 10 g/l, more preferably 0.5 - 5 g/l.

Preferably, when an iron transporter is present in said medium, said iron transporter is present in a concentration of 0.1-75 mg/ml, preferably 1-10 mg/l, more preferably about 5.5 mg/l.

Preferably, when insulin is present in said medium, said insulin is present in a concentration of 0.1 - 100 mg/l, preferably 5 - 75 mg/l, more preferably about 10 mg/l.

Preferably, when sodium selenite is present in said medium, said sodium selenite is present in a concentration of 0.01- 1000 µg/l, preferably 0.1 - 100 pg/l, more preferably 1 - 100 pg/l, most preferably about 6.7 µg/l.

Preferably, when a biogenic amine such as ethanolamine, putrescine, spermidine and/or spermine is present in said medium, said biogenic amine is present in a concentration of 0.01 - 100 mg/l, more preferably 0.1 - 10 mg/l, even more preferably 2-5 mg/l.

Preferably, when ascorbic acid or a derivative thereof such as L-ascorbic acid 2-phosphate is present in said medium, said ascorbic acid or said derivative thereof is present in a concentration of 0.01 - 10000 mg/l, preferably 1- 500 mg/l, more preferably about 50 mg/l.

Preferably, when somatotropin is present in said medium, said somatotropin is present in a concentration of 0.01 - 200 µg/l, preferably 0.1 - 20 µg/l, more preferably about 2 µg/l.

Preferably, when hydrocortisone is present in said medium, said hydrocortisone is present in a concentration of 0.01 - 1000 µg/l, preferably 1 - 500 pg/l, more preferably about 36 µg/l.

Preferably, when fibronectin is present in said medium, said fibronectin is present in a concentration of 0 - 1000 mg/l, preferably 0.1 - 100 mg/l, more preferably about 1 - 10 mg/l.

Preferably, when said one or more of PDGF, FGF, IGF, VEGF, HGF and IL-6 are present in said medium, said one or more of PDGF, FGF, IGF, VEGF, HGF and IL-6 are present in concentrations, respectively, of 1-100 µg/l, 1-100 µg/l, 0-1000 pg/l, 1-100 µg/l, 0.5-50 µg/l and 0.5-50 µg/l, more preferably about 10 pg/l, about 10 pg/l, 0-100 µg/l, about 10 µg/l, about 5 µg/l and about 5 µg/l, respectively. In certain embodiments, IGF can be absent in a serum-free medium as disclosed herein. Figures 2 and 4 show that the addition of growth factors and combination of growth factors is beneficial for cell growth.

Preferably, when said source of fatty acids is present in said medium, said source of fatty acids is present in a concentration of 0.01 - 100 mg/l, more preferably 0.1 - 10 mg/l, more preferably about 1 mg/l.

Preferably, when said protein hydrolysate is present in said medium, said protein hydrolysate is present in a concentration of 0 - 20 % (w/v), preferably 0.001 - 10 % (w/v), more preferably 0.01 - 1% (w/v), even more preferably 0 - 0.25% (w/v) or 0.01 - 0.25% (w/v), even more preferably 0-0.1% (w/v) or 0.01 - 0.1% (w/v), most preferably about 0.05% (w/v).

Preferably, when said one or more biogenic amine is present in said medium, said one or more biogenic amine, such as ethanolamine, putrescine, spermidine and/or spermine, is present in a concentration of 0 - 10 mg/l, more preferably 2 to 5 mg/l or 6-10 mg/l, most preferably about 2 or 5 mg/l.

Also disclosed herein is a method for producing a serum-free medium as disclosed herein, comprising the step of - adding the constituents or components of a serum-free medium as disclosed herein to a basal medium. In case one or more of the constituents or components of a serum-free medium as disclosed herein are already comprised in a basal medium, addition of said constituents or components of said serum-free medium to said basal medium can be omitted.

In a method for producing a medium as disclosed herein, said basal medium can be a liquid or powdered basal medium, preferably a liquid basal medium comprising DMEM and/or Ham's F12 medium, preferably DMEM and Ham's F12 medium, for instance in a ratio of 1:10 to 10:1, more preferably a 1:1 ratio, respectively. Alternatively, the basal medium may comprise RPMI or alpha-MEM.

In a method for producing a medium as disclosed herein, said constituents or components of a serum-free medium as disclosed herein include - an albumin; - a fibroblast growth factor (FGF); - ascorbic acid or a derivative thereof, an insulin, a somatotropin and a hydrocortisone; - a platelet-derived growth factor (PDGF), an insulin-like growth factor (IGF), a vascular endothelial growth factor (VEGF), an hepatocyte growth factor (HGF) and an interleukin 6 (IL-6); - a source of glucose (if not already comprised in said basal medium); - a source of glutamine; - a source of fatty acids; - a source of iron or an iron transporter; and - sodium selenite; and optionally a protein hydrolysate, a biogenic amine and/or an attachment factor, all of these components are preferably as disclosed herein, and are preferably in any one of the combinations as disclosed herein.

In another aspect, the invention provides a composition according to claim 9.

In a preferred embodiment of a composition of the invention, said progenitor cell is a (i) bovine muscle progenitor cell or a bovine fat (adipose tissue) progenitor cell or (ii) a bovine muscle tissue-derived progenitor cell or a bovine adipose tissue-derived progenitor cell.

In a preferred embodiment of a method for culturing a progenitor cell of the invention, said progenitor cell is a (i) bovine muscle progenitor cell or a bovine fat (adipose tissue) progenitor cell or (ii) a muscle tissue-derived progenitor cell or a bovine, ovine or porcine adipose tissue-derived progenitor cell.

In a preferred embodiment of a method for culturing a bovine progenitor cell of the invention, said bovine progenitor cell is a cell that is not genetically modified, or is a cell that is genetically modified.

In a preferred embodiment of a method for culturing a bovine progenitor cell of the invention, said bovine progenitor cell is a bovine myosatellite cell that is not genetically modified, or a bovine adipose-tissue (derived) progenitor cell that is not genetically modified.

In another aspect, the invention provides a use according to claim 11.

### DETAILED DESCRIPTION OF THE INVENTION

### Definitions

The term "serum-free", as used herein, includes reference to a culture medium that is formulated in the absence of serum such as human serum or bovine serum. A serum-free medium may contain serum proteins such as serum albumin by way of supplementation of said serum albumin to said medium. However, preferably, all components of said medium are animal-free. For instance, albumin is preferably recombinantly produced. The concentrations of the components of the serum-free medium as disclosed herein can be adjusted and optimized for the culturing and proliferation (expansion) of progenitor cells such as bovine progenitor cells. A serum-free medium can comprise a serum-free basal medium supplemented or not supplemented with other components such as a source of sugars, fatty acids, amino acids, vitamins, hormones, cytokines, growth factors or minerals. Preferably, in a serum-free medium as disclosed herein, said albumin, said FGF, said one or more cytokines and/or growth factors, said one or more vitamins and/or hormones, said basal medium, said source of glucose, said source of glutamine, said source of fatty acids, said source of iron or said iron transporter, said sodium selenite, said protein hydrolysate, said biogenic amine and/or said attachment factor are present in an effective amount for proliferation of a progenitor cell in culture.

The term "culturing", as used herein, includes reference to the propagation and/or proliferation (expansion) of progenitor cells such as bovine progenitor cells. In the context of the invention, this term preferably includes reference to the proliferation and therefore expansion of a bovine progenitor cell population. It should however be understood that a serum-free medium as disclosed herein can also be employed to proliferate (expand) other non-human, mammalian progenitor cells such as ovine and porcine progenitor cells. Therefore, any embodiment described herein in relation to bovine progenitor cells, is also applicable to ovine (such as sheep) and porcine (such as pig) progenitor cells, i.e. progenitor cells of ovine or porcine origin.

The terms "proliferation" and "expansion", as used herein, can be used interchangeably. These terms include reference to increasing the population size of progenitor cells, such as bovine progenitor cells, in cell culture, i.e. progenitor cells are generating other progenitor cells by cell proliferation. Such an expanded bovine progenitor cell population can subsequently be cultured in a differentiation media for differentiating said progenitor cells into muscle cells or adipose tissue that can be incorporated into a cell-culture based meat product for human consumption. However, first, before differentiation, sufficient amounts of progenitor cells, such as bovine progenitor cells, need to be produced by proliferation / expansion.

The term "progenitor cell", as used herein, includes reference to a cell that is committed to differentiate into a specific type of cell or to form a specific type of tissue. The term "progenitor cell" may include reference to multipotent stromal cells (mesenchymal stem cells) with the capacity for self-renewal and multipotential differentiation into *inter alia* myocytes (muscle cells) and adipocytes (fat cells).

Preferably, the progenitor cell is a muscle progenitor cell or a fat progenitor cell. More preferably, the progenitor cell is a bovine progenitor cells, more preferably a bovine muscle progenitor cell or a bovine adipose tissue (fat) progenitor cell.

A progenitor cell can be a tissue-derived progenitor cell, derived from a wildtype (e.g. domestic cow, sheep or pig) or a transgenic animal (e.g. transgenic cow, sheep or pig). The progenitor cell itself can be genetically modified or can be not genetically modified. For example, the progenitor cell can be an induced pluripotent stem cell (iPS) generated from a cell of bovine, ovine or porcine origin. Preferably, the progenitor cell is a muscle tissue- or adipose tissue(-derived) progenitor cell that is not genetically modified.

The term "bovine progenitor cells", as used herein, includes reference to a bovine cell that is committed to differentiate into a specific type of bovine cell or to form a specific type of bovine tissue. The term "bovine progenitor cell" may include reference to multipotent bovine stromal cells (mesenchymal stem cells) with the capacity for self-renewal and multipotential differentiation into *inter alia* myocytes (muscle cells) and adipocytes (fat cells). Preferably, the bovine progenitor cell is a bovine muscle progenitor cell or a bovine fat progenitor cell. A bovine progenitor cell can be a tissue-derived bovine progenitor cell, derived from a wildtype (e.g. domestic cow) or transgenic animal (e.g. transgenic cow). The bovine progenitor cell itself can be genetically modified or can be not genetically modified. For example, the bovine progenitor cell can be an induced pluripotent stem cell (iPS) generated from a bovine cell. Preferably, the bovine progenitor cell is a tissue-derived bovine progenitor cell that is not genetically modified.

The term "bovine", as used herein, includes reference to animals belonging to the family of *Bovidae,* including the genus *Bos.* The term "bovine", as used in aspects and embodiments described herein, may be replaced by the term "bovid". The term "bovid" can be used to refer to any animal in the family of *Bovidae.* The family of *Bovidae* includes bison, buffalo, antelopes, wildebeest, impala, gazelles, sheep, goats, muskoxen, and cattle (such as cows), including domestic cattle. Especially preferred bovine species are *Bos taurus* (cow).

The term "ovine", as used herein, includes reference to any animal that belongs to the genus of *Ovis,* which includes the species *Ovis aries.* The term includes reference to sheep, which can be a domesticated or a wild species.

The term "porcine", as used herein, includes reference to any animal in the family of *Suidae,* which includes the subfamily *Suinae* and the genus *Sus.* The term includes reference to pigs, which can be a domesticated or a wild species.

The term "muscle progenitor cell", or "muscle tissue-derived progenitor cell", as used herein, can be used interchangeably with the term "muscle stem cell" or "myogenic progenitor (cell)". These terms include reference to adult stem cells, present in muscle tissue such as skeletal muscle tissue, which are multipotent and which can self-renew and are capable of giving rise to muscle cells such as skeletal muscle cells. The term "muscle progenitor cell" can also be referred to as "muscle cell progenitor". A preferred muscle progenitor cell is a bovine muscle progenitor cell such as a bovine myosatellite cell. Preferably, the muscle progenitor cell, more preferably the myosatellite cell, is a (skeletal) muscle tissue-derived progenitor cell. Such a cell can be genetically modified or not genetically modified, preferably not genetically modified. Progenitor cells from the muscle can be isolated based on their positive expression of CD29 as previously described (Ding et al., Sci. Rep. 17(8): 10808 (2018)).

The term "myosatellite cell", as used herein, includes reference to a small multipotent cell and can be found in mature muscle tissue. Myosatellite cells are precursors to skeletal muscle cells, able to give rise to satellite cells or differentiated skeletal muscle cells. They are precursor cells that can be obtained from muscle tissue. They have the potential to provide additional myonuclei to their parent muscle fiber, or return to a quiescent state. More specifically, upon activation, satellite cells can re-enter the cell cycle to proliferate or differentiate into myoblasts. Myosatellite cells are generally located between the basement membrane and the sarcolemma of a muscle fibers. Myosatellite cells generally express a number of distinctive genetic markers. Most satellite cells express PAX7 and PAX3.

The term "fat progenitor cell" or "adipose tissue(-derived) (fat) progenitor cell", as used herein, can be used interchangeably with the terms "adipose stem cell", "stromal vascular fraction (SVF) cells" or "adipose tissue-derived stem cells (ADSCs)". These terms include reference to adult stem cells, for instance present in adipose tissue or in other tissues, which are multipotent and which can self-renew and are capable of giving rise to adipocyte-like cells such as adipose tissue cells. The term "fat progenitor cell" can also be referred to as "fat cell progenitor". As an example, bovine stromal vascular cells can be isolated from bovine subcutaneous fat tissue which is minced into small pieces and subjected to collagenase digestion. Stromal vascular cells can then be recovered by centrifugation and put in culture. In embodiments, the fat progenitor cell is a bovine adipose tissue-derived (fat) progenitor cell. Such a progenitor cell can be genetically modified or not genetically modified. In other embodiments, the fat (adipose tissue) progenitor cell is derived from a tissue other than adipose tissue.

The term "protein hydrolysate", as used herein, includes reference to a digest of a protein derived by acid, enzymatic or other hydrolysis of proteins, including vegetable proteins such as soy proteins, and comprises constituent amino acid residues as well as peptides of different sizes, representative of the source protein.

The term "biogenic amine", as used herein, includes reference to a biomolecule containing one or more amine groups. Included in this group of biogenic amines are monoamines and polyamines. Within the group of monoamines are included ethanolamine, and within the group of polyamines are included agmatine, cadaverine, putrescine, spermine and spermidine. Preferably, at least two biogenic amines are employed in a medium as disclosed herein, such as at least spermine and spermidine.

The term "iron", as used herein, includes reference to a salt containing iron such as ferric nitrate and ferrous sulfate.

The term "growth factor" as used herein, includes reference to a biomolecule, namely a protein regulating aspects of cellular function, such as survival and proliferation. Within the group of said proteins, FGF (such as FGF2 or basic FGF), IL-6, VEGF, IGF (such as IGF1), HGF and PDGF (such as PDGF-BB) are included. Preferably, the growth factors and/or hormones mentioned herein are of human origin, and are preferably recombinantly produced. In general, where reference is made to a growth factor, hormone or attachment factor that is a protein, such a protein can be a wildtype protein or a protein that is mutated or otherwise modified as compared to its wildtype equivalent, such as its human wildtype equivalent.

The term "attachment factor" as used herein includes reference to a structural protein, more preferably a glycoprotein. Within the group of glycoproteins fibronectin and laminin are included. Such attachment factors can be included in a serum-free medium as disclosed herein.

The term "source of', as used herein, includes reference to a medium component that is provided as a precursor of said medium component, or is provided as the medium component as such. The skilled person is well aware of suitable precursors for medium components as described herein. For instance, L-alanyl-L-glutamine or glutamine can be used as a source of glutamine, α-linolenic acid can be used as a source of fatty acids, and glucose can be used as a source of glucose.

### Serum-free medium of the disclosure

The disclosure provides a serum-free medium for culturing a bovine progenitor cell, comprising - an albumin; and - a fibroblast growth factor (FGF). Alternatively, the disclosure provides a serum-free medium for culturing a bovine progenitor cell, comprising - albumin; and - one or more growth factor(s) and/or cytokine(s) as described herein, preferably at least a fibroblast growth factor (FGF). The fibroblast growth factor (FGF) is preferably a human FGF, more preferably a human FGF basic (FGFb, also referred to as FGF2), even more preferably a human recombinant FGF basic, such as 233-FB from R&D Systems. Preferably, the albumin is a human albumin, such as a recombinant human albumin, for instance obtained from Biorbyt (orb419911). The albumin can be present in the medium in a concentration of 0.01 - 100 g/l, preferably 0.01 - 50 g/l, more preferably 0.1 - 10 g/l, more preferably 0.5 - 5 g/l such as about 5 g/l. The FGF can be present in the medium in a concentration of 0.1 - 1000 pg/l, preferably 1-100 pg/l, more preferably 5 - 50 pg/l, even more preferably about 10 µg/l.

Such a serum-free medium can be beneficially employed in the culturing, more specifically the propagation (expansion) of bovine progenitor cells. Amongst others, it was established that especially the presence of an albumin and a fibroblast growth factor (FGF) provides for improved bovine progenitor cell proliferation rates (Figure 4). It was established that with a serum-free culture medium as disclosed herein, growth rates comparable with serum-containing media could be achieved (Figure 3). Alternatively, a serum-free medium as disclosed herein can be employed in the culturing, more specifically the propagation (expansion), of ovine or porcine progenitor cells.

Preferably, a serum-free medium as disclosed herein further comprises a protein hydrolysate, preferably a vegetable protein hydrolysate, more preferably a soy protein hydrolysate. It was established that the presence of a protein hydrolysate increases bovine progenitor cell growth rates (Figure 5). One example of a soy protein hydrolysate that can be used is Soy Hydrolysate UF Solution 50X (Sigma Aldrich 58903C), which is an ultra-filtered enzymatic digest of soy. It is prepared with 250.0 g/L soy hydrolysate ultrafiltrate in cell culture grade water and is sterile filtered. The protein hydrolysate can be present in the medium in a concentration of 0 - 20 % (w/v), preferably 0.001 - 10 % (w/v), more preferably 0.01 - 1% (w/v), even more preferably 0 - 0.25% (w/v) or 0.01 - 0.25% (w/v), even more preferably 0 - 0.1% (w/v) or 0.01 - 0.1% (w/v), most preferably about 0.05% (w/v).

Preferably, a serum-free medium as disclosed herein further comprises a biogenic amine such a monoamine or a polyamine, including an ethanolamine, putrescine, spermidine and/or spermine. It was established that the presence of one or more biogenic amines increases growth rates both of muscle tissue-derived progenitor cells (Figure 5) and adipose-tissue-(derived) (fat) progenitor cells (Figure 6). Most preferably, the biogenic amine is ethanolamine or spermidine, or a combination of at least spermine and spermidine. The biogenic amine can be present in the medium in a concentration of 0 - 10 mg/l, more preferably 2 to 5 mg/l or 6-10 mg/l, most preferably about 2 or 5 mg/l. It is especially advantageous when both a protein hydrolysate as disclosed herein and a biogenic amine as disclosed herein are present in a serum-free medium as disclosed herein.

Preferably, a serum-free medium as disclosed herein further comprises one or more vitamins and/or hormones, preferably one or more of ascorbic acid or a derivative thereof such as L-ascorbic acid 2-phosphate, insulin, somatotropin and hydrocortisone. Preferably, the hormones (including insulin, somatotropin and hydrocortisone) are animal hormones, preferably mammalian hormones, more preferably human or bovine hormones, such as human hydrocortisone (H6909 from Sigma Aldrich), recombinant cow growth hormone protein (ab123464 from Abcam), and recombinant human insulin (91077C from Sigma Aldrich). More preferably, all of ascorbic acid or a derivative thereof such as L-ascorbic acid 2-phosphate (A8960), insulin, somatotropin and hydrocortisone are present in a serum-free medium as disclosed herein. Ascorbic acid or a derivative thereof such as L-ascorbic acid 2-phosphate can be present in the medium in a concentration of 0.01 - 10000 mg/l, preferably 1- 500 mg/l, more preferably about 50 mg/l. Insulin can be present in the medium in a concentration of 0.1 - 100 mg/l, preferably 5 - 75 mg/l, more preferably about 10 mg/l. Somatotropin can be present in the medium in a concentration of 0.01 - 200 µg/l, preferably 0.1 - 20 µg/l, more preferably about 2 µg/l. Hydrocortisone can be present in the medium in a concentration of 0.01 - 1000 µg/l, preferably 1 - 500 µg/l, more preferably about 36 µg/l. The one or more vitamins and/or hormones as disclosed herein are preferably present in a serum-free medium as disclosed herein in combination with said protein hydrolysate disclosed herein and said biogenic amine as disclosed herein.

Preferably, a serum free-medium as disclosed herein further comprises a source of glucose, a source of glutamine, and/or a source of iron or an iron transporter. A preferred source of glucose comprises glucose, a preferred source of glutamine comprises glutamine, and a preferred iron transporter is a transferrin. The source of glucose can be present in said medium in a concentration of 0.01 - 75 g/l, more preferably 0.1 - 4.5 g/l. The source of glutamine can be present in said medium in a concentration of 0.01 - 100 mM, more preferably 0.1 - 8 mM, such as about 2mM. The iron transporter can be present in the medium in a concentration of 0.1-75 mg/ml, preferably 1-10 mg/l, more preferably about 5.5 mg/l. Said source of glucose, said source of glutamine, and said source of iron or an iron transporter are preferably present in a serum-free medium as disclosed herein in combination with said protein hydrolysate as disclosed herein, said biogenic amine as disclosed herein, and said vitamins and/or hormones as disclosed herein.

Preferably, a serum free-medium as disclosed herein further comprises one or more cytokine and/or growth factor. Such cytokines and/or growth factor are preferably selected from the group formed by PDGF, IGF, VEGF, HGF, FGF and IL-6. All cytokines and/or growth factors are preferably human proteins, more preferably recombinant human proteins, such as recombinant human PDGF (e.g. 220-BB from R&D Systems), recombinant human IGF (e.g. 291-G1 from R&D Systems), recombinant human HGF (e.g. 294-HG from R&D Systems), recombinant human VEGF (e.g. 293-VE from R&D Systems), recombinant human FGF (e.g. 233-FB from R&D Systems), and recombinant human IL-6 (e.g. 206-IL from R&D Systems). Preferably, the PDGF is a PDGF-BB. Preferably, the IGF is an IGF1. Preferably, the IGF is a human IGF1. Preferably, the PDGF is a human PDGF-BB. Preferably, the FGF is a human FGF2, also referred to as bFGF. Preferably, all of PDGF, IGF, VEGF, HGF, FGF and IL-6 are present in a serum-free medium as disclosed herein. Alternatively, IL-6 and IGF are optional, and PDGF, VEGF, HGF and FGF are present in serum-free medium as disclosed herein. The PDGF can be present in the medium in a concentration of 0.1-1000 pg/l, preferably 1-100 pg/l, more preferably about 10 µg/l. The IGF can be present in the medium in a concentration of 0-10000 µg/l, preferably 0-1000 µg/l, more preferably about 0-100 µg/l. The VEGF can be present in the medium in a concentration of 0.1-1000 µg/l, preferably 1-100 pg/l, more preferably about 10 µg/l. The HGF can be present in the medium in a concentration of 0.05-100 µg/l, preferably 0.5-50 µg/l, more preferably about 5 µg/l. The IL-6 can be present in the medium in a concentration of 0.05-100 pg/l, preferably 0.5-50 pg/l, more preferably about 5 µg/l. The one or more cytokine and/or growth factor as disclosed herein are preferably present in a serum-free medium as disclosed herein in combination with said protein hydrolysate as disclosed herein, said biogenic amine as disclosed herein, said vitamins and/or hormones as disclosed herein and said source of glucose, said source of glutamine, and said source of iron or an iron transporter as disclosed herein.

Preferably, a serum free-medium as disclosed herein further comprises a source of fatty acids. A preferred source of fatty acids comprises α-linolenic acid (e.g. L2376 from Sigma Aldrich). The source of fatty acids can be present in the medium in a concentration of 0.01 - 100 mg/l, more preferably 0.1 - 10 mg/l, more preferably about 1 mg/l. The source of fatty acids as disclosed herein is preferably present in a serum-free medium as disclosed herein in combination with said protein hydrolysate as disclosed herein, said biogenic amine as disclosed herein, said vitamins and/or hormones as disclosed herein, said source of glucose, said source of glutamine, and said source of iron or an iron transporter as disclosed herein, and said one or more cytokine and/or growth factor as disclosed herein.

Preferably, a serum free-medium as disclosed herein further comprises a basal medium, preferably a liquid or powdered basal medium. Preferably, such a basal medium comprises (i) DMEM and/or Ham's F12 medium, preferably DMEM and Ham's F12 medium, for instance in a ratio of 1:10 to 10:1, more preferably a 1:1 ratio, respectively, (ii) RPMI medium and/or (iii) alpha-MEM (also referred to as Minimum Essential Medium α, MEM α, MEM alpha or AlphaMEM) medium. The skilled person is well aware of such media and knows how to obtain them. The basal medium as disclosed herein is preferably present in a serum-free medium as disclosed herein in combination with said protein hydrolysate as disclosed herein, said biogenic amine as disclosed herein, said vitamins and/or hormones as disclosed herein, said source of glucose, said source of glutamine, and said source of iron or an iron transporter as disclosed herein, said one or more cytokine and/or growth factor as disclosed herein and said source of fatty acids as disclosed herein. The skilled person understands that a basal medium may already by itself comprise some of the components or constituents of a serum-free medium as disclosed herein, which means that the addition of such components or constituents may be omitted in case they are already contained in the basal medium.

Preferably, a serum free-medium as disclosed herein further comprises sodium selenite. The sodium selenite (e.g. S5261 from Sigma Aldrich) as disclosed herein is preferably present in a serum-free medium as disclosed herein in combination with said protein hydrolysate as disclosed herein, said biogenic amine as disclosed herein, said vitamins and/or hormones as disclosed herein, said source of glucose, said source of glutamine, and said source of iron or an iron transporter as disclosed herein, said one or more cytokine and/or growth factor as disclosed herein, said source of fatty acids as disclosed herein and said basal medium as disclosed herein. Sodium selenite can be present in a serum-free medium in a concentration of 0.01- 1000 µg/l, preferably 0.1 - 100 µg/l, more preferably 1 - 50 or 1 -100 µg/l, most preferably about 6.7 µg/l.

Preferably, in a serum-free medium as disclosed herein, all the components are not obtained from animal material and are therefore animal-free (e.g. recombinantly produced).

Preferably, a serum-free medium as disclosed herein is a serum-free medium for proliferation of a progenitor cell.

The invention also provides a composition according to claim 9. The composition can be a cell culture.

The disclosure also provides a serum-free medium for culturing a bovine progenitor cell, comprising albumin (about 5 mg/ml), somatotropin (about 2 ng/ml), L-Ascorbic acid 2-phosphate (about 50 µg/ml), hydrocortisone (about 36 ng/ml), α-linolenic acid (about 1 µg/ml), insulin (about 10 µg/ml), transferrin (about 5.5 µg/ml), sodium selenite (about 0.0067 µg/ml), ethanolamine (about 2 µg/ml), L-alanyl-L-glutamine or glutamine (about 2mM), IL-6 (about 5 ng/ml), FGF2 also referred to as bFGF (about 10 ng/ml), IGF1 (about 100 ng/ml), VEGF (about 10 ng/ml), HGF (about 5 ng/ml), PDGF-BB (about 10 ng/ml) and DMEM / F12 basal medium. Said serum-free medium may optionally further comprise (i) a vegetable protein hydrolysate, such as a soy protein hydrolysate as described herein, and/or (ii) one more biogenic monoamine or polyamine as described herein, including one or more of ethanolamine, putrescine, spermidine and spermine, preferably at least spermidine and spermine. In addition, said serum-free medium may contain one or more attachment proteins, such as fibronectin.

### Methods for culturing progenitor cells

The invention also provides a method for culturing a bovine progenitor cell according to claim 1.

Preferably, the progenitor cell is a cell that is not genetically modified, or is a cell that is genetically modified. Preferably, said progenitor cell is a bovine, ovine or porcine progenitor cell, preferably a bovine, ovine or porcine muscle tissue-derived progenitor cell or a bovine, ovine or porcine adipose tissue-derived progenitor cell.

Exemplary culturing conditions for progenitor cells as described herein are as follows. Bovine progenitor cells, such as muscle tissue-derived progenitor cells or adipose tissue-derived progenitor cells, are seeded at a density of 3000-5000 cells/cm² in a serum-free medium as disclosed herein in an appropriate cell culture vessel. The aforementioned cell culture vessel may be pre-coated with a coating such as, but not limited to collagen. The cells can be passaged upon reaching 90% confluency. Briefly, the cells can be rinsed once with phosphate buffer saline (PBS, 20012027 from ThermoFischer Scientific) followed by the addition of trypsin (25200072 from ThermoFischer Scientific). Once the cells are detached, trypsin can be neutralised by the addition of trypsin inhibitor from *Glycine max* (T6522 from Sigma Aldrich), the cells collected into PBS and centrifuged at 350g. The supernatant can be aspirated and the cell pellet resuspended as needed. The cells can then be maintained in a 95% air/5% CO₂ humidified atmosphere at 37 °C.

### FIGURE LEGENDS

**Figure 1****.** The comparison of F10 and DMEM/F 12 media used as a base in a serum-free medium of the invention. Bovine muscle progenitor cells were cultured in collagen-coated 96 wells plate in basal medium F10 or DMEM/F 12 1:1 for 4 and 7 days (n=3). Cell numbers were measured with a proliferation assay MTS kit (Promega, CellTiter 96^{®} AQueous One Solution Cell Proliferation Assay (MTS)).
**Figure 2****.** The growth of bovine muscle progenitor cells when cultured in the serum-free medium for 4 days in different combinations of growth factors compared to the control serum based medium. The serum-free medium control contains DMEM/F12 1:1, supplemented with 1% penicillin/streptomycin/amphotericin (PSA), 2 mM L-alanyl-L-glutamine, 5 µg/ml bovine serum albumin, 75 ng/ml IL-6, and 5 µg/l FGF.
   The serum-containing medium control contains DMEM/F12 1:1 with 20% FBS.
   The concentrations of the growth factors tested were: IGF1 at 100 pg/l, HGF at 5 µg/l, VEGF at 10 pg/l, PDGF-BB at 10 µg/l.
**Figure 3****.** Long-term proliferation of bovine muscle progenitor cells in a serum-containing growth medium (Control GM) and a serum-free medium of the invention (SFM1). The tested serum-containing growth medium contains DMEM/F12 + 20% fetal bovine serum + 5 ng/ml bFGF + 1% Penicillin-Streptomycin-Amphotericin B) and the tested serum-free medium (SFM1) contains: albumin (5 mg/ml), somatotropin (2 ng/ml), L-Ascorbic acid 2-phosphate (50 µg/ml), hydrocortisone (36 ng/ml), α-linolenic acid (1 µg/ml), insulin (10 µg/ml), transferrin (5.5 µg/ml), sodium selenite (0.0067 µg/ml), ethanolamine (2 µg/ml), L-alanyl-L-glutamine or glutamine (2mM), IL-6 (5 ng/ml), FGF2 also referred to as bFGF (10 ng/ml), IGF1 (100 ng/ml), VEGF (10 ng/ml), HGF (5 ng/ml), PDGF-BB (10 ng/ml) and DMEM / F12 basal medium.
   The cells were cultured for several weeks in collagen coated tissue culture vessels and passed when 80%-90% confluent. At each passage, the cells were counted with a countess automated cell counter. Proliferation is shown in total population doublings (PDs).
**Figure 4****.** The growth of bovine muscle progenitor cells in the serum-free medium (control)) with different concentrations of several components. The cells were cultured in a collagen-coated 96 wells plate for 4 days (n=4) with different concentrations of the following components: recombinant human albumin (2, 1, 0,5 or 0 mg/ml), fibronectin from bovine plasma (5, 2, 1 or 0 µg/ml), recombinant human IL-6 (25, 10, 5, 2 or 0 ng/ml), recombinant human insulin-like growth factor 1 (25, 10, 5, 2 or 0 ng/ml) and recombinant fibroblast growth factor 2 (5, 2, 1 or 0 ng/ml). Cells were counted by the high content analyser ImageXpress Pico Automated Cell Imaging System. It is shown that the absence of albumin and FGF provides for highly reduced growth of bovine muscle progenitor cells.
**Figure 5****.** The effect of different concentrations of spermidine and a soy hydrolysate in the form of Soy Hydrolysate UF Solution on proliferation of bovine myosatellite cells. The cells were grown for 4 days (n=2) on collagen coated 96 wells plates with different concentrations of spermidine (0, 1, 2, 3, 4, 5 or 10 µg/ml) and different concentrations of soy hydrolysate (0, 0.05, 0.1, 0.15, 0.2 and 0.25 % (w/v). Cells counted by the high content analyser ImageXpress Pico Automated Cell Imaging System. It is shown that spermidine and soy protein hydrolysate enhance the cell growth rate.
**Figure 6**. Short-term culture comparison of the growth of adipose-tissue progenitor cells, i.e. stromal vascular fraction cells (from *Bos taurus*), in a serum-containing medium or serum-free medium with or without polyamine supplementation. The cells were seeded in 48 wells plates in a serum containing medium (DMEM/F12+10% fetal bovine serum + 2 ng/ml bFGF), serum-free medium (SFM1) or a serum-free medium (SFM1) supplemented with 1 ug/ml spermidine (SPMD) and 10 ug/ml spermine (SPMN). Following 6 days in culture, the cells were counted by the high content analyser ImageXpress Pico Automated Cell Imaging System.
**Figure 7****.** Short-term culture of satellite cells and adipose-tissue progenitor cells, i.e., stromal vascular fraction cells (SVF), all from *Bos taurus,* in a serum-free medium of the invention wherein the basal medium type was varied. The cells were seeded in collagen-coated 96 wells plate in a serum-free medium of the invention in which different basal media were included (i.e. RPMI medium, alpha-MEM medium, F12 medium, and DMEM/F 12 medium at 1:10, 1:1, and 10:1 ratios). Following 6 days in culture, the cells were counted by the high content analyser ImageXpress Pico Automated Cell Imaging System.

### EXAMPLES

### Example 1. Production of a serum-free medium of the invention

### Materials and methods

Bovine muscle progenitor cells were isolated from a bovine muscle tissue (*Bos taurus*) and sorted based on their positive expression of CD29 as previously described (Ding et al., Sci. Rep., 17(8): 10808 (2018)).

Cells were cultured in collagen-coated 96 well plates in basal medium DMEM/F12 1:1, supplemented with 1% PSA, 2 mM L-alanyl-L-glutamine, 5 µg/ml bovine serum albumin and 5 µg/l FGF (recombinant human protein FGF basic (FGFb), 233-FB from R&D Systems) for 5 days (n=3). This medium constitutes the serum-free control as indicated in the figure legend of Figure 2. For the serum-based control, the DMEM/F12 1:1 medium was supplemented with 20% FBS. For the tested conditions, the medium was supplemented with IGF1 (human recombinant IGF (291-G1 from R&D Systems)) at 100 µg/l, HGF (recombinant human HGF (294-HG from R&D Systems)) at 5 µg/l, VEGF (recombinant human VEGF (293-VE from R&D Systems)) at 10 µg/l and PDGF-BB (human recombinant PDGF (220-BB from R&D Systems)) at 10 pg/l, either alone or in combination. A two level, full factorial design of experiments (DoE) was performed to test the effect of different combinations of additional growth factors to the proliferation of bovine muscle progenitor cells. Cells were cultured at 37 °C, 5% CO₂ for 4 days. After 4 days they were fixed with paraformaldehyde 2% and stained with Hoechst staining. The number of cells per well was determined with the high content analyser ImageXpress Pico Automated Cell Imaging System. Statistical analysis was performed (JMP software) to investigate main effects and interaction effects between the growth factors tested (IGF1, HGF, VEGF and PDGF-BB).

### Results

The relative (expressed as a percentage) growth of the cells as compared to the serum containing control is shown in Figure 2. Statistical analysis of these results showed a significant positive effect of IGF1 and VEGF addition as well as a positive synergy of IGF1 with HGF. The serum free medium supplemented with IGF1 at 100 pg/l, HGF at 5 pg/l, VEGF at 10 pg/l, and PDGF-BB at 10 µg/l showed 1.5 fold increase in growth when compared to the serum contained control and a 4.7-fold increase of growth when compared to the serum-free based control (only containing 5 ng/mL FGF and 75 ng/ml IL-6 as growth factors).

### Example 2. Culturing a muscle progenitor cell in a medium of the invention.

### Materials and methods

Bovine muscle progenitor cells were isolated from a bovine muscle tissue *(Bos taurus*) and sorted based on their positive expression of CD29 as previously described (Ding et al., Sci. Rep., 17(8): 10808 (2018)).

Muscle progenitor cells were seeded at a density of 3000-5000 cells/cm² in the serum-free medium of invention (i.e. SFM1 medium) or in the serum containing medium (DMEM/F12 supplemented with 20% fetal bovine serum, 5 ug/ml bFGF and 2 mM L-alanyl-L-glutamine) in an appropriate collagen-coated cell culture vessel. The cells were passaged upon reaching 90% confluency. Briefly, the cells were rinsed once with phosphate buffer saline (PBS, 20012027 from ThermoFischer Scientific) followed by the addition of trypsin (25200072 from ThermoFischer Scientific). Once the cells were detached, trypsin was neutralised by the addition of trypsin inhibitor from *Glycine max* (T6522 from Sigma Aldrich), the cells collected into PBS and centrifuged at 350g. The supernatant was aspirated and the cell pellet resuspended as needed. The cells were maintained in a 95% air/5% CO₂ humidified atmosphere at 37 °C.

### Results

Muscle progenitor cells cultured in the serum-containing medium had an initial higher rate of growth that decreased with time, whereas the serum-free grown cells had a lower growth rate that remained relatively constant (more stable). With time, the total growth of cells in the two media was comparable. See Figure 3.

### Example 3. Culturing satellite cells and adipose-tissue progenitor cells in a serum-free medium of the invention with different basal media.

### Materials and methods

Satellite cells and adipose-tissue progenitor cells, e.g. stromal vascular fraction (SVF) cells, all from *Bos taurus,* were seeded in collagen-coated 96 wells plate and cultured for six days in a 95% air/5% CO₂ humidified atmosphere at 37 °C in a serum-free medium of the invention composed of albumin (5 mg/ml), somatotropin (2 ng/ml), L-Ascorbic acid 2-phosphate (50 µg/ml), hydrocortisone (36 ng/ml), α-linolenic acid (1 µg/ml), insulin (10 µg/ml), transferrin (5.5 µg/ml), sodium selenite (0.0067 µg/ml), ethanolamine (2 µg/ml), L-alanyl-L-glutamine or glutamine (2mM), IL-6 (5 ng/ml), FGF2 also referred to as bFGF (10 ng/ml), IGF1 (100 ng/ml), VEGF (10 ng/ml), HGF (5 ng/ml), PDGF-BB (10 ng/ml), with a different basal medium (i.e. RPMI medium (11875093, Thermo Fischer Scientific), alpha-MEM medium (12561056, Thermo Fischer Scientific), F12 medium (11765054, Thermo Fischer Scientific), DMEM/F12 1:1 medium, DMEM/F12 10:1 medium and DMEM/F12 1:10 medium) all made using F12 above and DMEM (A1443001, Thermo Fischer Scientific). Following 6 days in culture, the cells were counted by the high content analyser ImageXpress Pico Automated Cell Imaging System. The relative growth rate was calculated by dividing the number of cells in a certain medium by the number of days and normalizing to the control (growth in DMEM:F 12).

### Results

It was observed that advantageous growth (proliferation) rates of satellite cells and adipose-tissue progenitor cells were achieved with a serum-free medium of the invention comprising a spectrum of different basal media, and that growth (proliferation) rates of such cells do not depend on a specific basal medium (Figure 7).

## Claims

1. A method for culturing a bovine progenitor cell, comprising the step of:
culturing a bovine progenitor cell in a serum-free medium for culturing a bovine progenitor cell,
wherein said serum-free medium comprises
- an albumin;
- a fibroblast growth factor 2 (FGF2);
- an interleukin-6 (IL-6);
- an insulin-like growth factor 1 (IGF1); and
- an hepatocyte growth factor (HGF).

2. The method according to claim 1, wherein said serum-free medium further comprises
- one or more vitamins and/or hormones selected from the group consisting of ascorbic acid or a derivative thereof, an insulin, a somatotropin and a hydrocortisone.

3. The method according to claim 2, wherein said serum-free medium comprises an ascorbic acid or a derivative thereof.

4. The method according to claim 2 or claim 3, wherein said serum-free medium comprises a hydrocortisone.

5. The method according to any one of claims 1-4, wherein said serum-free medium further comprises:
- a PDGF and a VEGF.

6. The method according to any one of the preceding claims, wherein said serum-free medium further comprises
- a basal medium; preferably wherein the basal medium comprises (i) DMEM and/or Ham's F12 medium, preferably DMEM and Ham's F12 medium, for instance in a ratio of 1:10 to 10:1, more preferably a 1:1 ratio, respectively, (ii) RPMI medium and/or (iii) alpha-MEM medium;
- a source of glucose;
- a source of glutamine;
- a source of fatty acids; preferably wherein said source of fatty acids comprises α-linolenic acid;
- a source of iron or an iron transporter; and/or
- sodium selenite.

7. The method according to any one of the preceding claims, wherein said serum-free medium further comprises a protein hydrolysate, preferably a soy protein hydrolysate;
and/or wherein said serum-free medium further comprises a biogenic amine, preferably an ethanolamine, putrescine, spermidine and/or spermine.

8. The method according to any one of the preceding claims, wherein said serum-free medium is a serum-free medium for proliferation of a bovine progenitor cell.

9. A composition comprising a serum-free medium as defined in any one of the preceding claims and a bovine progenitor cell.

10. The method according to any one of claims 1-8, or the composition according to claim 9, wherein said bovine progenitor cell is a bovine muscle progenitor cell or a bovine fat (adipose tissue) progenitor cell.

11. Use of a serum-free medium in the production of a cell culture-based meat product for human consumption; wherein said serum-free medium is a serum-free medium for culturing a bovine progenitor cell, comprising
- an albumin;
- a fibroblast growth factor 2 (FGF2);
- an interleukin-6 (IL-6);
- an insulin-like growth factor 1 (IGF1); and
- an hepatocyte growth factor (HGF).

## Patentansprüche

1. Verfahren zur Kultivierung einer Rindervorläuferzelle, umfassend den Schritt von:
Kultivieren einer Rindervorläuferzelle in einem serumfreien Medium zur Kultivierung einer Rindervorläuferzelle,
wobei das serumfreie Medium umfasst:
- ein Albumin;
- einen Fibroblasten-Wachstumsfaktor 2 (FGF2);
- ein Interleukin-6 (IL-6);
- einen insulinähnlichen Wachstumsfaktor 1 (IGF1) und
- einen Hepatozyten-Wachstumsfaktor (HGF).

2. Verfahren nach Anspruch 1, wobei das serumfreie Medium weiterhin umfasst:
- ein oder mehrere Vitamine und/oder Hormone, ausgewählt aus der Gruppe bestehend aus Ascorbinsäure oder ein Derivat davon, einem Insulin, einem Somatotropin und einem Hydrocortison.

3. Verfahren nach Anspruch 2, wobei das serumfreie Medium eine Ascorbinsäure oder ein Derivat davon umfasst.

4. Verfahren nach Anspruch 2 oder Anspruch 3, wobei das serumfreie Medium ein Hydrocortison umfasst.

5. Verfahren nach einem der Ansprüche 1 bis 4, wobei das serumfreie Medium weiterhin umfasst:
- einen PDGF und einen VEGF.

6. Verfahren nach einem der vorhergehenden Ansprüche, wobei das serumfreie Medium weiterhin umfasst:
- ein Basalmedium; vorzugsweise wobei das Basalmedium (i) DMEM und/oder Hams F12-Medium, vorzugsweise DMEM und Hams F12-Medium, zum Beispiel jeweils in einem Verhältnis von 1:10 bis 10:1, mehr bevorzugt einem 1:1-Verhältnis, (ii) RPMI-Medium und/oder (iii) Alpha-MEM-Medium umfasst;
- eine Glucosequelle;
- eine Glutaminquelle;
- eine Quelle von Fettsäuren; vorzugsweise wobei die Quelle von Fettsäuren α-Linolensäure umfasst;
- eine Eisenquelle oder einen Eisentransporter und/oder
- Natriumselenit.

7. Verfahren nach einem der vorhergehenden Ansprüche, wobei das serumfreie Medium weiterhin ein Proteinhydrolysat, vorzugsweise ein Sojaproteinhydrolysat umfasst;
und/oder wobei das serumfreie Medium weiterhin ein biogenes Amin, vorzugsweise ein Ethanolamin, Putrescin, Spermidin und/oder Spermin umfasst.

8. Verfahren nach einem der vorhergehenden Ansprüche, wobei das serumfreie Medium ein serumfreies Medium zur Proliferation einer Rindervorläuferzelle ist.

9. Zusammensetzung, umfassend ein serumfreies Medium, wie in einem der vorhergehenden Ansprüche definiert, und einer Rindervorläuferzelle.

10. Verfahren nach einem der Ansprüche 1 bis 8 oder Zusammensetzung nach Anspruch 9, wobei die Rindervorläuferzelle eine Rindermuskelvorläuferzelle oder eine Rinderfettvorläuferzelle (Rinderfettgewebevorläuferzelle) ist.

11. Verwendung eines serumfreien Mediums in der Produktion eines zellkulturbasierten Fleischprodukts für den menschlichen Verzehr; wobei das serumfreie Medium ein serumfreies Medium zur Kultivierung einer Rindervorläuferzelle ist, umfassend
- ein Albumin;
- einen Fibroblasten-Wachstumsfaktor 2 (FGF2);
- ein Interleukin-6 (IL-6);
- einen insulinähnlichen Wachstumsfaktor 1 (IGF1) und
- einen Hepatozyten-Wachstumsfaktor (HGF).

## Revendications

1. Méthode de culture d'une cellule progénitrice bovine, comprenant l'étape suivante :
la mise en culture d'une cellule progénitrice bovine dans un milieu sans sérum de culture d'une cellule progénitrice bovine,
dans laquelle ledit milieu sans sérum comprend
- une albumine ;
- un facteur de croissance des fibroblastes de type 2 (FGF2) ;
- une interleukine-6 (IL-6) ;
- un facteur de croissance insulinomimétique de type 1 (IGF1) ; et
- un facteur de croissance des hépatocytes (HGF).

2. Méthode selon la revendication 1, dans laquelle ledit milieu sans sérum comprend en outre
- une ou plusieurs vitamines et/ou hormones sélectionnées dans le groupe consistant en l'acide ascorbique ou un dérivé de celui-ci, une insuline, une somatotropine et une hydrocortisone.

3. Méthode selon la revendication 2, dans laquelle ledit milieu sans sérum comprend un acide ascorbique ou un dérivé de celui-ci.

4. Méthode selon la revendication 2 ou la revendication 3, dans laquelle ledit milieu sans sérum comprend une hydrocortisone.

5. Méthode selon l'une quelconque des revendications 1 à 4, dans laquelle ledit milieu sans sérum comprend en outre :
- un PDGF et un VEGF.

6. Méthode selon l'une quelconque des revendications précédentes, dans laquelle ledit milieu sans sérum comprend en outre
- un milieu de base ; de préférence dans laquelle le milieu de base comprend (i) un milieu DMEM et/ou un milieu F12 de Ham, de préférence un milieu DMEM et un milieu F12 de Ham, par exemple à un rapport de 1:10 à 10:1, de manière davantage préférée un rapport de 1:1, respectivement, (ii) un milieu RPMI et/ou (iii) un milieu alpha-MEM ;
- une source de glucose ;
- une source de glutamine ;
- une source d'acides gras ; de préférence dans laquelle ladite source d'acides gras comprend de l'acide α-linolénique ;
- une source de fer ou un transporteur de fer ; et/ou
- du sélénite de sodium.

7. Méthode selon l'une quelconque des revendications précédentes, dans laquelle ledit milieu sans sérum comprend en outre un hydrolysat de protéines, de préférence un hydrolysat de protéines de soja ;
et/ou dans laquelle ledit milieu sans sérum comprend en outre une amine biogène, de préférence une éthanolamine, de la putrescine, de la spermidine et/ou de la spermine.

8. Méthode selon l'une quelconque des revendications précédentes, dans laquelle ledit milieu sans sérum est un milieu sans sérum pour la prolifération d'une cellule progénitrice bovine.

9. Composition comprenant un milieu sans sérum tel que défini dans l'une quelconque des revendications précédentes et une cellule progénitrice bovine.

10. Méthode selon l'une quelconque des revendications 1 à 8, ou composition selon la revendication 9, dans laquelle ladite cellule progénitrice bovine est une cellule progénitrice de muscle de bovin ou une cellule progénitrice de graisse de bovin (tissu adipeux).

11. Utilisation d'un milieu sans sérum dans la production d'un produit carné à base de culture cellulaire destiné à la consommation humaine ; dans laquelle ledit milieu sans sérum est un milieu sans sérum de culture d'une cellule progénitrice bovine, comprenant
- une albumine ;
- un facteur de croissance des fibroblastes de type 2 (FGF2) ;
- une interleukine-6 (IL-6) ;
- un facteur de croissance insulinomimétique de type 1 (IGF1) ; et
- un facteur de croissance des hépatocytes (HGF).
